# EUROPEAN PATENT APPLICATION

(11) **EP 2 292 624 A1**
(43) Date of publication of application: **09.03.2011**
(21) Application number: 09165882.3
(22) Date of filing: 20.07.2009
(51) Int. Cl.: C07D 495/04, A61K 31/551, A61P 25/18

(54) **Process for the purification of olanzapine**

(71) Applicant: LEK Pharmaceuticals d.d., 1526 Ljubljana (SI)
(72) Inventor: The designation of the inventor has not yet been filed

(57) **Abstract**

The present invention relates to a process for the purification of olanzapine, **characterized in that** said process comprises at least the following steps:
(A) providing a solution of olanzapine in at least one organic solvent,
(B) treating the solution obtained in step (A) with at least one non-acidic oxide adsorbent, and
(C) removing the said non-acidic oxide adsorbent from the solution obtained in step (B), in order to obtain a solution of olanzapine in a pure form,

to a mixture comprising olanzapine and at least one addition product of methylene chloride and olanzapine in an amount of 10 to 280 ppm, to a pharmaceutical formulation, to the use of said mixture and to a method of treating mental diseases and conditions, which comprises administering a therapeutically effective amount of said mixture in conjunction with a pharmaceutically acceptable diluent or carrier.

## Description

### FIELD OF INVENTION

The present invention relates to the field of organic chemistry and in particular to a process for the purification of olanzapine, wherein said process comprises a treatment of a solution of olanzapine with at least one non-acidic oxide adsorbent in order to obtain a solution of olanzapine in a pure form, from which olanzapine with reduced amount of impurities, in a specific case olanzapine and at least one addition product of methylene chloride and olanzapine in an amount of 10 to 280 ppm, is isolated. The present invention also relates to a pharmaceutical formulation, comprising this mixture.

### BACKGROUND OF THE INVENTION

Olanzapine is a pharmaceutical active substance from the group of antipsychotics, applicable for the treatment of different mental diseases and conditions, such as, for example, disorders of the central nervous system, schizophrenia, hallucination, acute mania, depression, and the like.

Chemically, it belongs to the group of the benzodiazepines and is 2-methyl-4-(4-methyl-1-piperazinyl)-10H-thieno[2,3-b][1,5]benzodiazepine (formula 1).

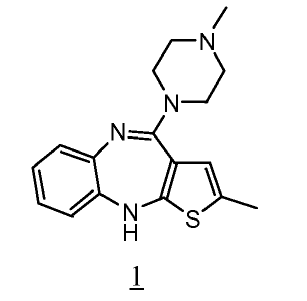

Olanzapine and analogues thereof are encompassed for the first time within a general formula in the patent GB 1,533,235 and specifically described in EP 454 436 B1. The patents disclose two different one-step processes for olanzapine preparation. The first described process is a reaction of 4-amino-2-methyl-10*H*-thieno[2,3-b][1,5]benzodiazepine hydrochloride with *N*-methylpiperazine in an organic solvent, such as anisole, toluene, dimethyl formamide or dimethyl sulfoxide, preferably at a temperature from 100 to 150°C to yield olanzapine (Scheme 1).

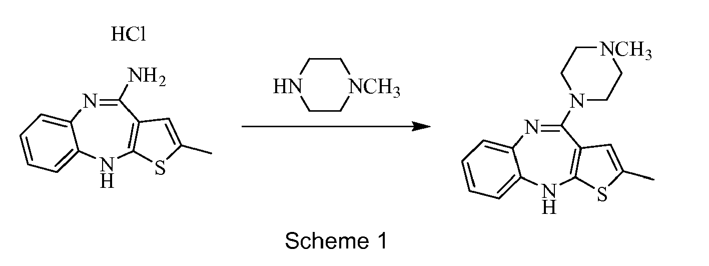

The second process disclosed in EP 454 436 B1 is the reaction of *N*-methylpiperazine with methyl-2-(2-aminoanilino)-5-methylthiophene-3-carboxylate in the presence of titanium tetrachloride (Scheme 2).

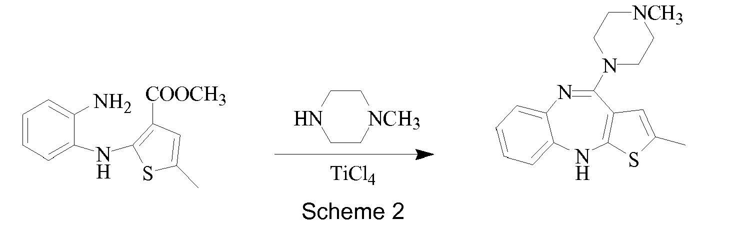

The same patent also mentions the formation of acid addition salts of olanzapine and their potential use as intermediates in olanzapine purification process and for a pharmaceutical use.

As disclosed in EP 454 436 B1 and US equivalent US 5,229,382, olanzapine obtained according to the first synthesis (Scheme 1) is purified by recrystallization from acetonitrile, whereas olanzapine prepared according to the second route (Scheme 2) is further purified by column chromatography on Florisil^{®} and recrystallization from acetonitrile. This purification procedure lacks on industrial applicability.

Some other synthetic approaches for the preparation of olanzapine describe two steps for creating 4-methylpiperazinyl side chain (Scheme 3). Firstly, 4-amino-2-methyl-10H-thieno [2,3-b][1,5] benzodiazepine hydrochloride reacts with piperazine to yield 2-methyl-4-(1-piperazinyl)-10H-thieno[2,3-b][1,5]benzodiazepine (i.e. *N*-desmethylolanzapine; Bioorganic & Medicinal Che-mistry Letters, Vol. 7, No. 1, pp. 25-30, 1997), then the methyl group is introduced either by reductive *N*-methylation (using formaldehyde and metal boron hydride - WO 04/000847) or by nucleophylic substitution reaction with methyl iodide (WO 05/090359). The two-step approach reduces the dark colour appearance but it does not solve the problem of purification from similar by-products.

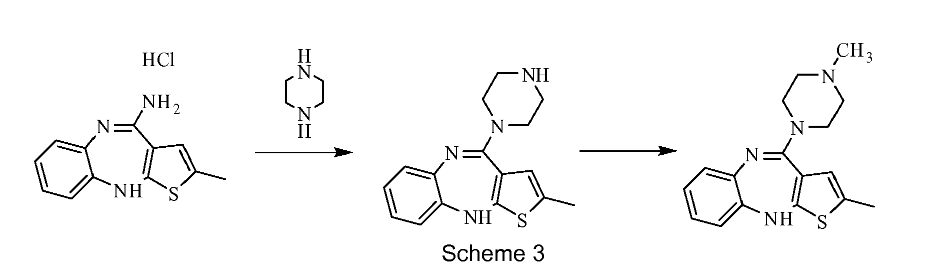

It is well known to a skilled person that most chemical reactions are not completely finished, may be reversible or are driven simultaneously with some other parallel reactions. Starting materials or side reaction products are usually found as impurities in the isolated main product which should therefore be further purified. The simplest way of purification includes various recrystallization and precipitation procedures which are usually less effective if the impurities have physico-chemical properties very similar to the main product.

In the case where olanzapine is prepared according to the one step processes disclosed in EP 454 436 B1, the starting material, 4-amino-2-methyl-10*H*-thieno[2,3-b][1,5]-benzodiazepine, is found as an impurity in the final product olanzapine.

In the case of preparation of olanzapine via the two-step process, as disclosed also in the patent application WO 04/000847, the presence of 4-amino-2-methyl-10*H*-thieno[2,3-b][1,5]-benzodiazepine hydrochloride is not critical but various other similar compounds could be found as impurities, such as 4-(4-formylpiperazinyl)-2-methyl-10*H*-thieno[2,3-b][1,5]-benzo diazepine and *N*-desmethylolanzapine. In the case of preparation of olanzapine by a two-step process with methyl iodide, an overmethylated *N,N*-dimethylpiperazinium analogue can be formed.

A further undesired impurity which accompanies olanzapine is obtained when olanzapine compound is dissolved in methylene chloride. It is so called olanzapine-CM, being (*E*)-1-(chloromethyl)-1-methyl-4-(2-methyl-10*H*-benzo[b]thieno[2,3-e][1,4]diazepin-4-yl)piperazin-1-ium chloride). Olanzapine-CM is formed by alkylation of olanzapine with methylene chloride in methylene chloride solution, for example during evaporation of methylene chloride before the crystallization (Scheme 4).

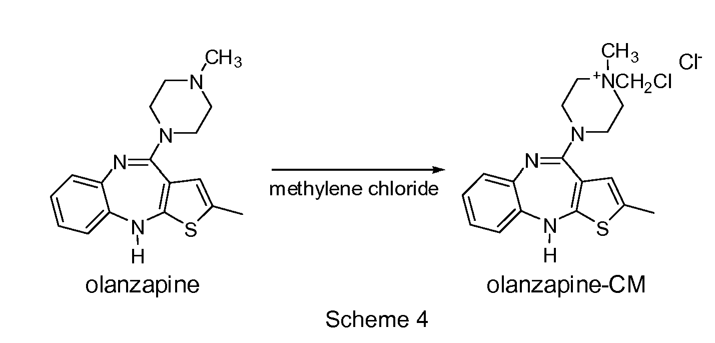

According to the Regulatory Toxicology and Pharmacology 44, pp. 198-211, 2006, classification of potential genotoxic impurities, olanzapine-CM is a potential genotoxic substance due to its R-CH₂-Cl structural element, which is known to be involved in reactions with DNA.

For all impurities that have a thienobenzodiazepine ring system as a part of the molecule skeleton and because it represents a great part of the molecule, said ring system is crucial for the similarity of physico-chemical properties of said impurities compared to olanzapine.

Different salts and crystal forms of an active pharmaceutical ingredient are an important tool for modulating pharmacokinetic properties but can be also a tool for purification.

WO 04/089313 discloses olanzapine acid salts, solvates and co-crystals and their use as active pharmaceutical ingredients in formulations. The preparation of fumaric, maleic and malonic acid addition salts of olanzapine is disclosed in WO 04/089313. Olanzapine acid addition salts disclosed in this application exhibit specific aqueous solubility from 50 µg/ml to 100 mg/ml.

WO 05/090359 discloses a method for the purification of olanzapine which has on one hand been prepared by the one-step-process according to Scheme 1, and on the other hand by the process according to Scheme 3, by preparing an addition salt of at least one carboxylic salt, purification of said salt and transfer into purified olanzapine.

Neutral olanzapine can be isolated in various crystal forms, hydrates and other solvates. However crystal forms I and II are the most often mentioned as an active pharmaceutical ingredient. Both forms were first disclosed in EP 733 635 alleging that form II is thermodynamically more stable than form I which had been prepared already by the basic patent procedures (EP 454 436 B1).

Crystal Growth & Design, Vol. 3, No. 6, pp. 897-907, 2003 discloses anhydrates and hydrates of olanzapine.

Olanzapine form I is thermodynamically less stable but it can possess specific kinetic properties which can be applied in designing a final dosage form. Many procedures are known how to prepare it but a person skilled in the art can soon find that the use of methylene chloride is unavoidable to develop a repeatable process. Because this solvent is used in the final step of preparation of olanzapine form I, previous purification methods cannot prevent the presence of the impurity olanzapine-CM in the final product.

WO 03/101997 A1 discloses a process for the preparation of a pure olanzapine form I by an addition of methyl-piperazine to the hydrochloride salt of the corresponding benzodiazepine derivative. The purification of olanzapine is conducted by recrystallization. Olanzapine-CM is not disclosed as a disturbing side product which can be removed by recrystallization.

WO 02/18390 discloses a process for the preparation of hydrates of olanzapine and the conversion thereof into crystalline forms of olanzapine by recrystallization from methylene dichloride. It is not disclosed that essentially pure olanzapine can be obtained by the removal of olanzapine-CM.

WO 04/056833 A1 discloses a process for the preparation of essentially pure olanzapine by removing olanzapine-CM, which is obtained from a solution of olanzapine in methylene chloride, by treating this solution with SiO₂, followed by the removal of SiO₂. According to the examples, olanzapine is obtained having a purity of 99.92 %, whereas olanzapine-CM is present in an amount of 0.05 %, corresponding to 500 ppm. But SiO₂ is acidic, so it adsorbs well also the basic olanzapine what leads to considerable losses of the mother compound.

Olanzapin-CM has to be removed in order to obtain essentially pure olanzapine with a high purity which can be further used in pharmaceutical applications. It has been found that olanzapine cannot be efficiently separated from its highly related impurities, in particular from olanzapine-CM, using repeated crystallizations of crude olanzapine. It would therefore be desirable to develop a purification process, in order to provide pharmaceutically acceptable pure and discoloured olanzapine, in particular to provide pharmaceutically acceptable pure olanzapine, being essentially free of the olanzapine-CM impurity. A further object of the present invention is to provide a process for the purification of olanzapine which can also and preferably be conducted in a large scale synthesis.

### DETAILED DESCRIPTION OF THE INVENTION

According to the present invention, the above mentioned objects can be solved by the process for the purification of olanzapine, **characterized in that** said process comprises at least the following steps:
(A) providing a solution of olanzapine in at least one organic solvent,
(B) treating the solution obtained in step (A) with at least one non-acidic oxide adsorbent, and
(C) removing the said non-acidic oxide adsorbent from the solution obtained in step (B), in order to obtain a solution of olanzapine in a pure form.

According to the present invention, a non-acidic oxide adsorbent means a non-acidic adsorbent or an adsorbent with a reduced acidity, which is selected from the group of metal or semi metal (metalloid) oxides. Hydroxy groups of partially hydrated forms of such non-acidic metal or semi metal oxides are in contrast to the free SiO₂ partially or completely neutralized, substituted or coated. An adsorbent of such type expresses outwards a neutral or slightly basic character. An adsorbent with a reduced acidity means a primarily acidic oxide adsorbent in which acidic character is not completely neutralized but the adsorbent still possesses the adsorption properties according to the present invention.

Preferably, in the process according to the present invention, non-acidic oxide adsorbent is selected from the group consisting of Al₂O₃, silica gel-NH₂, i. e. aminoalkyl substituted SiO₂, TiO₂, MgO, ZnO and mixtures thereof.

The oxide adsorbents of the present invention have surprisingly a weaker adsorption of olanzapine than silica but retain a strong adsorption of quaternary salts. A greater difference of adsorption properties between olanzapine and quaternary olanzapine impurities which are in silica too close enables a more efficient removal of more polar impurities like olanzapine-CM which consequently increases the yield of the parent compound and reduces the impurity level.

These objects can further be solved by a mixture comprising olanzapine and at least one addition product of methylene chloride and olanzapine in an amount of 10 to 280 ppm, by a pharmaceutical formulation, comprising said mixture, by the use of at least one oxide adsorbent chosen from the group consisting of Al₂O₃, silica gel-NH₂, TiO₂, MgO, ZnO and mixtures thereof in the purification of olanzapine, by the use of said mixture for the preparation of a medicament for the treatment of mental diseases and conditions, by the use of said mixture for the preparation of a pharmaceutical formulation together with at least one pharmaceutically acceptable ingredient, by a method of treating mental diseases and conditions, such as disorders of the central nervous system, schizophrenia, hallucination, acute mania, depression, and the like, which comprises administering a therapeutically effective amount of said mixture in conjunction with one or more pharmaceutically acceptable diluents or carriers.

The single steps of the process according to the present invention are explained in detail in the following:

### Step (A):

Step (A) of the process according to the present invention comprises providing a solution of olanzapine in at least one organic solvent.

Basically, olanzapine can exist in several different morphologic forms, for example Form I, Form II, Form III, Form IV, Form V, solvates, hydrates, which are commonly known to the skilled artisan (see for example Crystal Growth & Design, Vol. 3, No. 6, pp. 897-907, 2003*)*, Form I and Form II are the most important polymorphs for the pharmaceutical use.

According to the present invention, olanzapine in any isolated solid form, which is dissolved in at least one organic solvent, can be treated by the process according to the present invention, in order to reduce impurities, but in a preferred embodiment, providing a solution of olanzapine according to step (A) of the process according to the present invention, means taking a solution from an olanzapine preparation step such as using
- extracts from reaction and isolation mixtures,
- filtrates before or after crystallization,
- concentrates of evaporation,
- residues of evaporation in a form of oil, foam or amorphous solid followed by dissolving,
- olanzapine salts as isolable intermediates followed by alkalizing and removing anion.

In a preferred embodiment, in step (A) of the process according to the present invention, a solution of olanzapine is provided which is directly obtained from the addition salts, for example olanzapine oxalate, after preparation, which explained in detail below.

Olanzapine which is used in step (A) of the process according to the present invention can be obtained by any process for the preparation of olanzapine which are known to the skilled artisan. In preferred embodiments, olanzapine is obtained according to GB 1,533,235, either by the reaction of 4-amino-2-methyl-10*H*-thieno[2,3-b][1,5]benzodiazepine hydrochloride with *N*-methylpiperazine in an organic solvent such as anisole, toluene, dimethyl formamide or dimethyl sulfoxide, preferably at a temperature from 100 to 150 °C to yield olanzapine according to the scheme 1 as mentioned above, or by the reaction of *N*-methylpiperazine with methyl-2-(2-aminoanilino)-5-methylthiophene-3-carboxylate in the presence of titanium tetrachloride according to the scheme 2 as mentioned above.

A further particularly preferred process for the preparation of olanzapine which can be introduced into step (A) of the process according to the present invention is the reaction of 4-amino-2-methyl-10H-thieno[2,3-b][1,5]benzodiazepine hydrochloride with piperazine to produce *N*-desmethylolanzapine according to the scheme 3 as mentioned above. This synthesis route is published in Bioorganic & Medicinal Chemistry Letters, Vol. 7, No. 1, pp. 25-30, 1997. In order to yield olanzapine, the obtained *N*-desmethylolanzapine is reacted with a methylation agent, being for example methyl iodide, dimethyl sulfate, trimethyl phosphate, under basic conditions. Different amines may be used, such as triethylamine, diisopropylamine, dicyclohexylamine, ethyldiisopropylamine and diazabicyclooctane, or strong bases of alkaline or alkaline-earth metals, such as hydroxides, hydrides or alcoholates, for example sodium hydride, calcium hydride, potassium t-butoxide, sodium or potassium hydroxide, as well as other inorganic bases, such as potassium or sodium carbonate. Methylation is, for example, conducted at a temperature of -40 to +20 °C, preferably from -30 to 0 °C, in a polar solvent, like THF, methylene chloride, optionally with the addition of a "co-solvent", for example dimethylacetamide, dimethyl formamide, dimethyl sulfoxide, N-methyl-2-pyrrolidinone, 1,3-dimethyl-3,4,5,6-tetrahydro-2(1*H*)-pyrimidinone or a mixture of two or more of them. After the methylation of *N*-desmethylolanzapine under said conditions, preferably olanzapine of a bright yellow colour without any brown or green tinge is obtained which does not require subsequent removal of unwanted colour.

As previously disclosed, *N*-desmethylolanzapine which remains in the reaction mixture after the completion of the methylation reaction, should preferably be removed from olanzapine. Thus the reaction mixture which is obtained after the methylation of *N*-desmethylolanzapine can be first extracted with organic solvents, such as ethers, e. g. diethyl ether; esters, e. g. ethyl acetate; or preferably chlorinated organic solvents, such as methylene chloride and chloroform. The remaining *N*-desmethylolanzapine is converted to acetyl derivative with acetanhydride. After that, oxalic acid is added. Olanzapine is crystallized and filtered off; acetyl olanzapine stays in the mother liquor.

As a final step in the preparation of olanzapine which is preferably introduced into step (A) of the process according to the present invention, olanzapine can optionally be purified through the transformation to an acid addition salt thereof. In this occurrence, an organic acid is added to the reaction mixture, such as sulfonic acids or carboxylic acid, preferably oxalic, fumaric or benzoic acid, and the like, more preferably oxalic acid, to form an olanzapine acid addition salt which can be precipitated out of the mixture after cooling and can be filtered off. The aqueous solubility of the prepared oxalic acid addition salt of olanzapine is approximately up to about 800 mg/mL.

The olanzapine acid addition salt can be afterwards transformed to a pure olanzapine in crystal forms I or II by dissolving the olanzapine acid addition salt in water and the pH is adjusted to about 1-5, preferably to 2 by the addition of hydrochloric acid. Charcoal is added to the resulting solution. After stirring the mixture for about 5 minutes, charcoal is filtered off and the cake is washed with water. The filtrate and wash water are combined followed by the addition of a low boiling organic solvent, such as diethyl ether, methylene chloride, chloroform, ethyl acetate, and the like, preferably methylene chloride. The treatment of the reaction mixture with charcoal at a pH of about 2 corresponds to the final purification of olanzapine and allows to get rid of the dimer of olanzapine being piperazine 1,4-bis-4-yl-(2-methyl)-10*H*-thieno-[2,3-b][1,5]benzodiazepine.

The next step is the addition of a base, preferably an inorganic base, such as ammonia, K₂CO₃, KOH, NaOH, Na₂CO₃, LiOH, Ca(OH)₂ and the like, more preferably NaOH, to obtain a pH of about 7-11, preferably a pH of about 9-10. After the desired pH is obtained, the mixture can be extracted with a low boiling organic solvent whereby different solvents can be used, such as ethers, e.g. diethyl ether; chlorinated hydrocarbons, e.g. methylene chloride, chloroform; esters, e.g. ethyl acetate; and the like, preferably methylene chloride. After the extraction, the organic solvent can be partly removed by evaporation and the residual mixture can be cooled to about -20 °C to about 0 °C, preferably about -15°C to about -5 °C, and olanzapine crystal form I is obtained. The obtained olanzapine contains a very low amount of said dimer (piperazine 1,4-bis-4-yl-(2-methyl)-10*H*-thieno-[2,3-b][1,5] benzodiazepine), such as lower than about 0.05 %. This embodiment to obtain a solution of olanzapine, which can be used in step (A) of the process according to the present invention, is preferred.

In another embodiment of the present invention, a process for producing olanzapine crystal form I is obtained via a different route. In order to obtain crude olanzapine, the reaction predefined in EP 454 436 B1 is used. The reaction comprises reacting of *N*-methyl piperazine with 4-amino-2-methyl-10*H*-thieno[2,3-b][1,5]-benzodiazepine hydrochloride. The reaction is carried out in the presence of a high boiling organic solvent, such as dimethyl sulfoxide, dimethylacetamide, butanol, dimethylformamide, toluene, xylene, ethylbenzene, anisole and the like, preferably dimethyl sulfoxide, at a temperature of about 80 to 150 °C, preferably about 115 to 130°C. The resulting solution that contains crude olanzapine after treatment with water is extracted from the obtained solution with an organic solvent whereby different solvents can be used, for example ketones, such as methylisobutylketone; chlorinated hydrocarbons, such as methylene chloride and chloroform, preferably dichloromethane. Thereafter an organic acid, such as carboxylic acid, for example oxalic, fumaric, benzoic acid or sulfonic acids and the like is added to form an acid addition salt of olanzapine. The olanzapine acid addition salt can be filtered off, and optionally dissolved in water and extracted out of the solution with an organic solvent, such as ketones, e.g. methylisobutylketone; chlorinated hydrocarbons, e.g. methylene chloride and chloroform. Afterwards the acid addition salt is isolated by evaporation of the solvent. The obtained olanzapine acid addition salt is transformed to olanzapine by first dissolving it in water and the pH is adjusted to about 1-5, preferably to 2, by the addition of hydrochloric acid. Charcoal is added to the resulting solution. After stirring the mixture for about 5 minutes, charcoal is filtered off and the cake is washed with water. The filtrate and wash water are combined, followed by the addition of a low boiling organic solvent, such as diethyl ether, methylene chloride, chloroform, ethyl acetate and the like, preferably methylene chloride. The next step is the addition of a base, preferably an inorganic base, such as ammonia, K₂CO₃, KOH, NaOH, Na₂CO₃, LiOH, Ca(OH)₂ and the like, more preferably NaOH, to obtain a pH of about 7-11, preferably about 9-10. After the desired pH is obtained, the mixture can be extracted with a low boiling organic solvent, whereby different solvents can be used, such as ethers, e.g., diethyl ether; chlorinated hydrocarbons, e.g., methylene chloride, chloroform; esters, e.g. ethyl acetate; and the like, preferably methylene chloride. After the extraction, the organic solvent can be partly removed by evaporation and the residual mixture can be cooled to about -20 °C to about 0 °C, preferably about -15 °C to about -5 °C, and crystals are precipitated and filtered off to give olanzapine form I after drying.

A further optional process consists of the treatment of the remaining solution from any crystallization step of olanzapine with the process according to the present invention. The solution which remains after olanzapine is filtered off, can be directly treated with an organic acid, such as carboxylic acid, for example oxalic, fumaric, benzoic or sulfonic acids and the like. The precipitation of thus formed olanzapine acid addition salt takes place immediately or said remaining solution can be first concentrated by evaporating off the solvents and the solution can be further diluted by other solvents or a mixture of solvents being more suitable for the acid addition salt precipitation, preferably by the addition of the mixture of methylene chloride and methanol. The obtained olanzapine acid addition salt can further be transformed to a pure olanzapine by the above described procedure.

Preferably, according to step (A) of the process of the present invention, an olanzapine solution is obtained directly in the medium which follows the process of isolation from the reaction mixture, preferably from the step of converting an olanzapine addition salt to a free base which is then extracted into the organic solvent.

In a further embodiment according to step (A) of the process of the present invention, a solid olanzapine, preferably crystalline, most preferably in the crystal form I, is dissolved in at least one organic solvent in order to provide the mentioned solution.

In general, all organic solvents can be used in step (A) of the process, according to the present invention. In a preferred embodiment of the process according to the present invention, the organic solvent in step (A) is chosen from the group consisting of ethers, e. g. diethyl ether; chlorinated hydrocarbons, e. g. methylene chloride, chloroform; esters, e. g. ethyl acetate, and the like; and mixtures thereof. In a particularly preferred embodiment, methylene chloride is used as the solvent in step (A) of the process according to the present invention.

The solution which is provided in step (A) of the process according to the present invention comprises at least olanzapine. Because the process according to the present invention is essentially conducted in order to remove the side-product olanzapine-CM from olanzapine, olanzapine-CM is also present in the solution which is provided in step (A). Olanzapine-CM may be present in olanzapine used in step (A) of the process according to the present invention in an amount of at least 300 ppm, probably at least 600 ppm, more probably at least 1000 ppm.

In a further embodiment, the solution which is provided in step (A) of the process according to the present invention may further comprise other olanzapine impurities like dimeric or polar impurities, such as the overmethylated product and other potential quaternized olanzapine products, in addition to olanzapine.

In the solution which is provided in step (A) of the process according to the present invention, olanzapine is present in a concentration of in general 2 to 200 g/L, preferably 10 to 150 g/L, particularly preferably 20 to 100 g/L. In a preferred embodiment of the process according to the present invention, said concentration is provided by removing of the solvent from more diluted solutions.

Step (A) of the process according to the present invention can be conducted in any reactor which is suitable and known to a person having ordinary skill in the art, for example glass flasks, beakers, glassline reactors, reactors made of stainless steel, and the like.

Step (A) of the process according to the present invention can be conducted at any temperature at which olanzapine is preferably completely dissolved in the at least one organic solvent, for example at 0 to 120 °C, preferably at 10 to 50 °C, most preferably at room temperature. The temperature under which step (A) of the process according to the present invention is conducted is further depending on the organic solvent that is used, especially on the boiling point thereof. In the preferred case of methylene chloride, step (A) of the process according to the present invention is conducted at room temperature.

After step (A), a solution of olanzapine in at least one organic solvent is obtained, which is preferably directly transferred to step (B) of the process according to the present invention.

Optionally, before step (B) is conducted, the solution which is obtained in step (A) can be additionally treated
- by adsorbents of the state of the art in order to reduce the amount of less polar impurities, preferably by active charcoal and/or
- by decanting or transferring the solution to another vessel if a sediment or a vessel wall coating appears and/or
- by reducing the solvent volume to obtain a concentration of olanzapine of 10 to 200 g/L, preferably to 20 to 150 g/L, particularly preferably to 40 to 100 g/L.

### Step (B):

Step (B) of the process according to the present invention comprises treating the solution obtained in step (A) with at least one non-acidic oxide adsorbent. In a preferred embodiment of the process according to the present invention, the non-acidc oxide adsorbent is chosen form the group consisting of Al₂O₃, silica gel-NH₂, TiO₂, MgO, ZnO and mixtures thereof.

In a very preferred embodiment of step (B) of the process according to the present invention, the solution obtained in step (A) is treated with Al₂O₃. According to the present invention, any Al₂O₃, which is known to the skilled artisan, can be used in step (B), for example alpha-, beta- and/or gamma-Al₂O₃, neutral or basic, in a particle size suitable for a smooth filtration or effective filling of pads or columns. In a particularly preferred embodiment, Al₂O₃ basic, particle size 0.05 to 0.15 mm, pH 9.5 is used in step (B) of the process according to the present invention. Al₂O₃ which is used in step (B) can be obtained by processes which are known to the skilled artisan. In addition, Al₂O₃ is commercially available.

In the sense of the present invention, "treating" means bringing the solution of olanzapine which has been provided in step (A) of the process according to the present invention into contact with the at least one non-acidic oxide adsorbent, being preferably chosen from the group consisting of Al₂O₃, silica gel-NH₂, TiO₂, MgO, ZnO and mixtures thereof.

In general, all methods which are known to the skilled artisan can be employed in order to conduct step (B) of the process according to the present invention.

In a preferred embodiment of the process according to the present invention, step (B) is conducted
(a) by the addition of at least one non-acidic oxide adsorbent to the solution,
(b) by filtration of the solution through a pad of said non-acidic oxide adsorbent and/or
(c) by eluting of the solution through a column of said non-acidic oxide adsorbent.

In a first possible embodiment (a), step (B) of the process according to the present invention is conducted by the addition of the oxide adsorbent to the solution. In this embodiment, the oxide adsorbent is added to the solution that has been provided in step (A).

The amount of the oxide adsorbent is in general not restricted. In embodiment (a) of step (B), the weight ratio of the oxide adsorbent and olanzapine being present in the solution is 0.001 to 10, preferably 0.01 to 5, more preferably 0.2 to 5.

After the addition of the oxide adsorbent to the solution, a slurry is obtained. Preferably, this slurry is stirred, in order to bring the dissolved olanzapine and the oxide adsorbent into contact. Suitable apparatus for stirring are known to the skilled artisan, for example a reactor comprising a stirring bar, stirrer blades, impeller. As an alternative or in addition to stirring, ultra-sonic may be applied to the slurry, in order to obtain a good distribution of the oxide adsorbent in the slurry.

In embodiment (a), the slurry comprising at least olanzapine and at least one oxide adsorbent is in general stirred for a time which is long enough to remove all olanzapine-CM from the solution, for example 1 to 60 minutes, preferably 2 to 20 minutes, more preferably 3 to 15 minutes. After completion of stirring, the slurry comprising the solution and the oxide adsorbent are preferably directly transferred to step (C) of the process according to the present invention.

In a second possible embodiment (b) of step (B) of the process according to the present invention the solution provided in step (A) is treated in step (B) by filtration of the solution through a pad of said non-acidic oxide adsorbent.

Methods of filtration of a solution through a solid compound are, in general, known to the skilled artisan. For example, a pad of the non-acidic oxide adsorbent can be placed into a tube or a column having for example a filter and/or a frit at the lower end, which is preventing that the non-acidic oxide adsorbent is washed off the tube or the column. Suitable tubes or columns are known to the skilled artisan, for example glass columns which are already known from column chromatography, e. g. short columns. The diameter and height of the tube or column depend on the amount of the solution which is to be treated, and can be chosen by the skilled artisan. In a preferred embodiment, the tube or the column is arranged vertically, in order to force the solution through the non-oxidic oxide adsorbent, preferably chosen from the group consisting of Al₂O₃, silica gel-NH₂, TiO₂, MgO, ZnO and mixtures thereof, by gravity. Instead of or in addition to gravity, a pressure above the atmospheric pressure can be applied, for example by nitrogen or argon.

The weight ratio of the metal oxide adsorbent and olanzapine being present in the solution which is treated in the method (b) of step (B) is in general 1 to 10, preferably 2 to 8, more preferably 3 to 7. The skilled artisan does know which diameter and which height of the pad of the non-acidic oxide adsorbent, which is depending on the tube or column which is used, shall be used in order to obtain the mentioned weight ratios.

In general, the method (b) of step (B) of the process is conducted by simple pouring of the solution onto the non-acidic oxide adsorbent pad.

In a further possible embodiment of step (B) of the process according to the present invention, both preferred methods of "treating" are conducted subsequently. In one embodiment, firstly, the oxide adsorbent is added to the solution obtained in step (A), and, secondly, the solution obtained therefrom is filtrated through the pad of the non-acidic oxide adsorbent. In another embodiment, firstly, the solution obtained in step (A) is filtered through the pad of at least one non-acidic oxide adsorbent, and secondly, the non-acidic oxide adsorbent is added to the solution obtained after filtration.

Method (c) of step (B) of the process according to the present invention comprises eluting of the solution through a column of said non-acidic oxide adsorbent.

In particular, method (c) is a preferred embodiment of method (b) as explained above. Therefore the details and preferred embodiments of this embodiment of method (b) also apply to method (c).

### Step (C):

Step (C) of the process according to the present invention comprises removal of the non-acidic oxide adsorbent from the solution obtained in step (B), in order to obtain a solution of olanzapine in a pure form.

If step (B) of the process according to the present invention is conducted according to method (a), step (C) is conducted by common methods for separating solids from liquids, for example filtration, centrifugation, decantation, etc., that are known to the skilled artisan. In this case, step (C) is preferably conducted by filtration.

If step (B) of the process according to the present invention is conducted according to the method (b) or (c), step (C) is conducted *in situ*, meaning that the oxide adsorbent is separated from the solution by the use of a filter and/or frit at the lower end of the tube or column which is used in step (B). In this preferred embodiment, steps (B) and (C) of the process according to the present invention are both conducted in one apparatus, being for example a tube or a column having a filter and/or a frit at the lower end.

After step (C), a solution of olanzapine in a pure form is obtained.

In order to have solid olanzapine in a pure form, the solvent has to be removed from the solution that is obtained in step (C). This can be conducted by common methods being known to a skilled artisan, for example evaporation of the solvent, crystallization of solid olanzapine, preferably followed by filtration, or preferably by a partial evaporation of the solvent followed by filtration.

The evaporation of the solvent is in general conducted by heating the solution to a temperature which is approximately as high as the boiling point of the solvent. For example, if methylene chloride, having a boiling point of 40 °C at the atmospheric pressure, is used as the solvent, the evaporation can be conducted at a temperature of 35 to 60 °C, preferably at 40 to 55 °C. For other solvents, the skilled artisan does know how to choose the right temperature. Evaporation can be conducted at atmospheric pressure or, preferably, at a pressure below the atmospheric pressure, for example below 800 mbar, preferably below 600 mbar, more preferably below 400 mbar. If the evaporation is conducted at a pressure below atmospheric pressure, the temperature can be decreased accordingly. Evaporation is in general conducted with a common apparatus or by a common evaporation technique directly from a reactor or any other type of the reaction or separation vessel.

In a preferred embodiment of the present invention, pure solid olanzapine is obtained by crystallization. In general, methods for crystallization of solid compounds from their solution are known to the skilled artisan. In a preferred embodiment, crystallization is obtained by cooling the solution to a temperature below the room temperature, for example to -20 to 10 °C. This can be conducted by common methods, for example by using an ice/salt-bath or a refrigerator. In a preferred embodiment, the concentration of olanzapine in the solution is increased prior to cooling, for example by partly removing the solvent by evaporation.

Pure olanzapine which is obtained by the present process can be analyzed by common methods, for example by using HPLC, IR, XRD, ¹H- and ¹³C-NMR, which are known to the skilled artisan.

Purity of olanzapine which is obtained by the present process is at least 99.5 %, preferably at least 99.6 %, more preferably 99.7 % or more. Purity is preferably acquired by HPLC.

Olanzapine which is obtained by the present process shows the advantage that the undesired side product olanzapine-CM has been removed, essentially completely. Olanzapine which is obtained by the process according to the present invention comprises olanzapine-CM in an amount of 10 to 280 ppm, preferably of 12 to 200 ppm, more preferably 15 to 150 ppm, most preferably 20 to 100 ppm.

The process according to the present invention gives rise to olanzapine in an essentially pure form, preferably the process according to the present invention gives rise to olanzapine in any crystal form which isolation procedure in any step includes methylene chloride in an essentially reduced amount of at least one additional product of methylene chloride and olanzapine, most preferably it gives rise to olanzapine form I which isolation procedure includes methylene chloride in the last step in an essentially reduced amount of at least one additional product of methylene chloride and olanzapine.

The present invention also relates to a mixture comprising olanzapine and at least one additional product of methylene chloride and olanzapine in an amount of 10 to 280 ppm, preferably of 12 to 200 ppm, more preferably 15 to 150 ppm, most preferably 20 to 100 ppm. In this mixture, the addition product of methylene chloride and olanzapine is preferably present in an amount of more than 10 ppm.

The mixture obtained by the processes of the present invention is suitable for pharmaceutical use in any pharmaceutical formulation.

The mixture obtained by the processes of the present invention is suitable for conversion to other pharmaceutically acceptable salts, preferably to olanzapine pamoate.

The present invention further relates to a pharmaceutical formulation, comprising the mixture according to the present invention and at least one pharmaceutically acceptable ingredient.

Suitable ingredients are, for example, pharmaceutically acceptable carriers. In making the compositions of the invention, conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

Depending on the method of administration, the compositions may be formulated as tablets, capsules, injection, solutions for parenteral use, suspensions or elixirs for oral use or suppositories or patches / films.

The mixture comprising olanzapine prepared by the processes of the present invention and the pharmaceutical formulation can accordingly be used for the prevention and/or treatment of different mental diseases and conditions, such as, for example, disorders of the central nervous system, schizophrenia, hallucination, acute mania, depression, and the like.

According to the present invention, there is provided a method of treating mental diseases and conditions, such as, for example, disorders of the central nervous system, schizophrenia, hallucination, acute mania, depression, and the like, which comprises administering a therapeutically effective amount of the mixture according to the present invention in conjunction with a pharmaceutically acceptable diluent or carrier.

The mixture of the invention will normally be administered orally or by injection or patch / film and, for this purpose, it is usually employed in the form of a pharmaceutical formulation. The mixture which is obtained from the process according to the present invention and excipients may be formulated into pharmaceutical formulations according to methods known in the art.

The present invention further relates to the use of at least one oxide adsorbent chosen from the group consisting of Al₂O₃, silica gel-NH₂, TiO₂, MgO, Zn in the purification of olanzapine.

The present invention further relates to the use of a mixture for the preparation of a medicament for the treatment of mental diseases and conditions.

The present invention further relates to the use of a mixture for the preparation of a pharmaceutical formulation together with at least one pharmaceutically acceptable ingredient.

The present invention further relates to a method of treating mental diseases and conditions, such as disorders of the central nervous system, schizophrenia, hallucination, acute mania, depression, and the like, which comprises administering a therapeutically effective amount of the mixture according to the present invention in conjunction with a pharmaceutically acceptable diluent or carrier.

### EXAMPLES

### Example 1 - Synthesis of olanzapine oxalic salt

A solution of 12.0 g of *N*-desmethylolanzapine in 90 mL of THF and 36 mL of dimethylacetamide (DMAc) is cooled to approx. -20 °C. At -20 °C, 8.19 g of diisopropylamine is added to the solution and afterwards, 8.14 g of methyl iodide is added over 30 min. After stirring the reaction mixture for 65 minutes at -20 °C, 6.4 mL of concentrated hydrochloric acid in 50 mL of water and a solution of 6.36 g of thiourea in 50 mL of water is added and the reaction mixture is stirred for 15 minutes at 20 °C. Then THF is evaporated off and 120 mL of methylene chloride is added and the pH is adjusted to 8.6 with a 40 % water solution of NaOH. After the separation of the phases, the water phase is washed twice with 60 and 30 mL of methylene chloride. The organic phases are combined and 380 mg of acetic anhydride is added and the mixture is stirred for 5 minutes. Then 6.20 g of oxalic acid in 24 mL of methanol is added within 15 minutes. The resulting suspension is stirred for about 1 hour at approx. 20 °C and afterwards 1 hour at approx. 0 °C. The product is isolated by filtration, washed with 100 mL of methylene chloride and dried for 15 hours at 25 °C in vacuo. Yield: 15 g (93 %).

### Example 2 - Formation of olanzapine form I

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. After the solution is concentrated in vacuo to the volume of 15 mL, the solution is immediately cooled on an ice/salt bath. The resulting suspension is stirred for 15 minutes, and then olanzapine is isolated by filtration. The wet cake is washed with 3 mL of methylene chloride of the temperature of -20 °C. The product is dried for four hours at 100 °C in vacuo.

| | |
|---|---|
| HPLC-Purity: | 99.9 % |
| olanzapine-CM | 380 ppm |
| IR | Form I |
| XRD | Form I |

### Example 3 - Formation of olanzapine form I (scale up)

24 kg of olanzapine oxalate is dissolved in 240 L of water and the pH of the solution is adjusted to 2.0 by the addition of 6 N HCl. To the resulting clear solution of olanzapine oxalate, 2.4 kg of charcoal is added. After stirring for 5 minutes, the charcoal is filtered off and the cake is washed with 10 L of water. The filtrate and wash water are combined and after the addition of 300 L of methylene chloride, the pH of the combined mixture is adjusted to 9.0 by the addition of a 40 % water solution of NaOH. After stirring for 15 minutes, the layers are separated and the water phase is extracted twice with 50 L of methylene chloride. The organic layers are combined and washed twice with 100 L of water. After the solution is concentrated in vacuo to the volume of 50 L, the solution is immediately cooled to -15 °C. The resulting suspension is stirred for 30 minutes, then olanzapine is isolated by filtration. The wet cake is washed with 10 L of methylene chloride of the temperature of -20 °C. The product is dried for 10 hours at 100 °C in vacuo.

| | |
|---|---|
| HPLC-Purity: | 99.7 % |
| olanzapine-CM | 1000 ppm |
| IR | Form I |
| XRD | Form I |

### Example 4 - Formation of olanzapine form I (Al₂O₃ fluidized bed adsorption)

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. After 0.2 g of Al₂O₃ is added and the methylene chloride suspension is stirred for 5 minutes, Al₂O₃ is filtered off and the methylene chloride solution is concentrated to the volume of 15 mL. The solution is immediately cooled on an ice/salt bath. The resulting suspension is stirred for 15 minutes, and then olanzapine is isolated by filtration. The wet cake is washed with 3 mL of methylene chloride of the temperature of -20 °C. The product is dried for four hours at 100 °C in vacuo.

| | |
|---|---|
| HPLC-Purity: | 99.9 % |
| olanzapine-CM | 214 ppm |
| IR | Form I |
| XRD | Form I |

### Example 5 - Formation of olanzapine form I (Al₂O₃ fluidized bed adsorption)

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. Afterwards, the methylene chloride solution is concentrated to 30 mL and 0.2 g of Al₂O₃ is added. After stirring for 5 minutes, Al₂O₃ is filtered off and the methylene chloride solution is concentrated to the volume of 15 mL. The solution is immediately cooled on an ice/salt bath. The resulting suspension is stirred for 15 minutes, and then olanzapine is isolated by filtration. The wet cake is washed with 3 mL of methylene chloride of the temperature of -20 °C. The product is dried for four hours at 100 °C in vacuo.

| | |
|---|---|
| HPLC-Purity: | 99.9 % |
| Olanzapine-CM | 321 ppm |
| IR | Form I |
| XRD | Form I |

### Example 6 - Formation of olanzapine form I (Al₂O₃ fluidized bed adsorption)

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. Then the methylene chloride solution is concentrated to 30 mL and 1 g of Al₂O₃ is added. After stirring for 5 minutes, Al₂O₃ is filtered off and the methylene chloride solution is concentrated to the volume of 15 mL. The solution is immediately cooled on an ice/salt bath. The resulting suspension is stirred for 15 minutes, and then olanzapine is isolated by filtration. The wet cake is washed with 3 mL of methylene chloride of a temperature of -20 °C. The product is dried for four hours at 100 °C in vacuo.

| | |
|---|---|
| HPLC-Purity: | 99.9 % |
| olanzapine-CM | 138 ppm |
| IR | Form I |
| XRD | Form I |

### Example 7 - Formation of olanzapine form I (Al₂O₃ short column adsorption)

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. Then the methylene chloride solution is concentrated to 30 mL and filtered through 20 g of Al₂O₃ (h = 2 cm). After the methylene chloride solution is concentrated to the volume of 15 mL, the solution is immediately cooled on an ice/salt bath. The resulting suspension is stirred for 15 minutes, and then olanzapine is isolated by filtration. The wet cake is washed with 3 mL of methylene chloride of the temperature of-20 °C. The product is dried for four hours at 100 °C in vacuo.

| | |
|---|---|
| HPLC-Purity: | 99.9 % |
| olanzapine-CM | 162 ppm |
| IR | Form I |
| XRD | Form I |

### Example 8 - Formation of olanzapine form I (Al₂O₃ short column adsorption)

5 g of olanzapine oxalate is dissolved in 50 mL of water and the pH of the solution is adjusted to 2.0 by the addition of 6 N HCl. To the resulting clear solution of olanzapine oxalate, 0.5 g of charcoal is added. After stirring for 5 minutes, the charcoal is filtered off and the cake is washed with 10 mL of water. The filtrate and wash water are combined and after the addition of 60 mL of methylene chloride, the pH of the combined mixture is adjusted to 9.0 by the addition of a 40 % water solution of NaOH. After stirring for 5 minutes, the layers are separated and the water phase is extracted twice with 10 mL of methylene chloride. The organic layers are combined and washed twice with 20 mL of water. Afterwards, the methylene chloride solution is concentrated to 30 mL and filtered through 20 g of Al₂O₃ (h = 5 cm). After the methylene chloride solution is concentrated to the volume of 15 mL, the solution is immediately cooled on an ice/salt bath. The resulting suspension is stirred for 15 minutes, and then olanzapine is isolated by filtration. The wet cake is washed with 3 mL of methylene chloride of the temperature of-20 °C. The product is dried for four hours at 100 °C in vacuo.

| | |
|---|---|
| HPLC-Purity: | 99.9 % |
| olanzapine-CM | 73 ppm |
| IR | Form I |
| XRD | Form I |

**Table 1. Comparison and overview of beneficial effect of Al₂O₃ treatment**

| Ex. | Scale | Treatment | Mass (Al₂O₃) [g] | Al₂O₃ pad height [cm] | CM assay [ppm] |
|---|---|---|---|---|---|
| 2 | 5 g | none | / | / | 380 |
| 3 | 24 kg | none | / | / | 1000 |
| 4 | 5 g | fluidized bed adsorption | 0.2 | / | 214 |
| 5 | 5 g | fluidized bed adsorption (concentrated to 30 mL) | 0.2 | / | 321 |
| 6 | 5 g | fluidized bed adsorption (concentrated to 30 mL) | 1.0 | / | 138 |
| 7 | 5 g | short column adsorption (concentrated to 30 mL) | 20 | 2 | 162 |
| 8 | 5 g | short column adsorption (concentrated to 30 mL) | 20 | 5 | 73 |

**Table 2. As comparative data, olanzapine obtained by processes according to the prior art comprises the following amounts of olanzapine-CM:**

| No. | prior art | amount of olanzapine-CM |
|---|---|---|
| 1 | WO 2005/090359, lab. scale | 300 - 400 ppm |
| 2 | WO 2005/090359, industrial scale | 800 - 2000 ppm |
| 3 | WO 2004/056833 | <0.15% |

## Claims

1. A process for the purification of olanzapine, **characterized in that** said process comprises at least the following steps:
(A) providing a solution of olanzapine in at least one organic solvent,
(B) treating the solution obtained in step (A) with at least one non-acidic oxide adsorbent, and
(C) removing the said non-acidic oxide adsorbent from the solution obtained in step (B), in order to obtain a solution of olanzapine in a pure form.

2. The process according to claim 1, wherein the said organic solvent in step (A) is chosen from the group consisting of ethers, chlorinated hydrocarbons, esters and mixtures thereof.

3. The process according to claim 1 or 2, wherein step (B) is conducted
(a) by the addition of at least one non-acidic oxide adsorbent to the solution,
(b) by filtration of the solution through a pad of said non-acidic oxide adsorbent and/or
(c) by eluting of the solution through a column of said non-acidic oxide adsorbent.

4. The process according to any of claims 1 to 3, wherein the non-acidic oxide adsorbent is chosen from the group consisting of Al₂O₃, silica gel-NH₂, TiO₂, MgO, ZnO, and mixtures thereof, preferably Al₂O₃.

5. The process according to any of claims 1 to 4, wherein in the solution which is provided in step (A) olanzapine is present in a concentration of 20 to 250 g/L.

6. The process according to claim 5, wherein said concentration is provided by removing of the solvent from more diluted solutions.

7. A mixture comprising olanzapine and at least one addition product of methylene chloride and olanzapine in an amount of 10 to 280 ppm.

8. A pharmaceutical formulation, comprising a mixture according to claim 7 and at least one pharmaceutically acceptable ingredient.

9. Use of at least one non-acidic oxide adsorbent chosen from the group consisting of Al₂O₃, silica gel-NH₂, TiO₂, MgO, ZnO and mixtures thereof in the purification of olanzapine.

10. Use of the mixture according to claim 7 for the preparation of a medicament for the treatment of mental diseases and conditions.

11. Use of the mixture according claim 7 for the preparation of a pharmaceutical formulation together with at least one pharmaceutically acceptable ingredient.

12. A method of treating mental diseases and conditions, such as disorders of the central nervous system, schizophrenia, hallucination, acute mania, depression, and the like, which comprises administering a therapeutically effective amount of the mixture according to claim 7 in conjunction with a pharmaceutically acceptable diluent or carrier.
